# EUROPEAN PATENT APPLICATION

(11) **EP 4 749 269 A1**
(43) Date of publication of application: **27.05.2026**
(21) Application number: 24862549.3
(22) Date of filing: 16.08.2024
(51) Int. Cl.: G01N 21/65, G01N 21/05, G01N 21/49

(54) **INFORMATION PROCESSING APPARATUS, OPERATION METHOD FOR INFORMATION PROCESSING APPARATUS, AND OPERATION PROGRAM FOR INFORMATION PROCESSING APPARATUS**

(30) Priority: 05.09.2023 JP 2023143805
(71) Applicant: FUJIFILM Corporation, Tokyo 106-8620 (JP)
(72) Inventor: SUGITA, Yui, Ashigarakami-gun, Kanagawa 258-8577 (JP)
(74) Representative: Hoffmann Eitle
(86) International application number: PCT/JP2024/029195
(87) International publication number: WO 2025/052895

(57) **Abstract**

An information processing apparatus that predicts a state of a target component in a suspension in which biological molecules are dispersed as components in a liquid, based on spectral data obtained by measuring electromagnetic waves emitted from the suspension includes a processor, in which the processor is configured to use a state prediction model calibrated with standard spectral data obtained from a standard suspension in a standard measurement environment, acquire target spectral data obtained from a target suspension in which the state of the target component is unknown, in a target measurement environment different from the standard measurement environment, acquire a correction coefficient derived by comparing the standard spectral data with reference spectral data that corresponds to the target spectral data and that is obtained from the standard suspension in the target measurement environment, correct the target spectral data with the correction coefficient to generate corrected target spectral data, and apply the corrected target spectral data to the state prediction model so that the state prediction model predicts the state of the target component.

## Description

### BACKGROUND OF THE INVENTION

### 1. Field of the Invention

The technology of the present disclosure relates to an information processing apparatus, an operation method of an information processing apparatus, and an operation program of an information processing apparatus.

For example, a manufacturing process for biopharmaceuticals containing biological molecules such as proteins, including monoclonal antibodies, as active ingredients is known. In such a manufacturing process, a suspension in which various components including the active ingredients are dispersed in a liquid is often produced. It is important to monitor a state (for example, a protein concentration or a protein aggregate concentration) of a target component (for example, a protein or a protein aggregate) among the components in the suspension in order to successfully lead the ongoing manufacturing process.

As the technology of predicting the state of the target component, the following technology has attracted attention because it poses less concern about contamination. That is, this technology is to measure electromagnetic waves emitted from the suspension, such as Raman scattering light, and apply spectral data such as Raman spectral data obtained by the measurement to a state prediction model such as a multivariate analysis or a machine learning model, thereby causing the state prediction model to predict the state of the target component.

In addition, JP1997-089775A (JP-H09-089775A) discloses a technology of correcting variations in Raman spectral data due to fluctuations in intensity of excitation light from a light source. In addition, JP2022-552876A discloses a technology of suppressing variations in a plurality of Raman spectral data by performing derivative transformation and standard normal variate (SNV) transformation on the plurality of Raman spectral data.

### SUMMARY OF THE INVENTION

In the manufacturing process of the biopharmaceuticals, conditions are first set using relatively small-scale equipment, and, after the condition setting is completed, the process is shifted to relatively large-scale equipment, with the scale of the equipment used gradually increasing. As the scale of the equipment increases, a measurement environment of electromagnetic waves also changes in various ways, such as changing a flow cell that is a measuring device for stably measuring electromagnetic waves.

In a case where the measurement environment of the electromagnetic waves is changed, the spectral data may be different even for suspensions of the same composition. Therefore, although a prediction result for the state of the target component should be the same for the suspensions of the same composition, different prediction results are obtained because the spectral data differs depending on the measurement environment. In short, the reliability of the prediction result for the state of the target component may be reduced due to the change in the measurement environment of the electromagnetic waves.

One embodiment according to the technology of the present disclosure provides an information processing apparatus, an operation method of an information processing apparatus, and an operation program of an information processing apparatus that are capable of obtaining a highly reliable prediction result for a state of a target component in a suspension even in a case where a measurement environment of electromagnetic waves is changed.

According to the present disclosure, there is provided an information processing apparatus that predicts a state of a target component in a suspension in which biological molecules are dispersed as components in a liquid, based on spectral data obtained by measuring electromagnetic waves emitted from the suspension, the information processing apparatus comprising: a processor, in which the processor is configured to use a state prediction model calibrated with standard spectral data obtained from a standard suspension in a standard measurement environment, acquire target spectral data obtained from a target suspension in which the state of the target component is unknown, in a target measurement environment different from the standard measurement environment, acquire a correction coefficient derived by comparing the standard spectral data with reference spectral data that corresponds to the target spectral data and that is obtained from the standard suspension in the target measurement environment, correct the target spectral data with the correction coefficient to generate corrected target spectral data, and apply the corrected target spectral data to the state prediction model so that the state prediction model predicts the state of the target component.

It is preferable that the correction coefficient be derived based on a ratio of intensity values of the standard spectral data and the reference spectral data corresponding to the component in the standard suspension.

It is preferable that the correction coefficient be derived by excluding a ratio of intensity values corresponding to a measuring device for the electromagnetic waves.

It is preferable that the measuring device be a flow cell having a flow path through which the suspension flows and an optical system for introducing the electromagnetic waves, and the intensity value corresponding to the measuring device be an intensity value corresponding to the optical system.

It is preferable that the standard measurement environment and the target measurement environment differ in any of whether a measuring device for the electromagnetic waves is used or a type of the measuring device.

It is preferable that the measuring device be a flow cell having a flow path through which the suspension flows and an optical system for introducing the electromagnetic waves.

It is preferable that there be a plurality of the target measurement environments, and the plurality of target measurement environments use different types of the flow cells.

It is preferable that the correction coefficient be stored in advance in a storage unit for each of the plurality of target measurement environments, and the processor be configured to receive a designation of the type of the flow cell used, and acquire the correction coefficient corresponding to the designation by reading out the correction coefficient from the storage unit.

It is preferable that the flow cells differ in at least one of a distance between an incident surface of the optical system for the electromagnetic waves and a wall surface of the flow path facing the incident surface, a reflectivity of the wall surface, or a focal length of the optical system.

It is preferable that the state prediction model be calibrated with the corrected target spectral data.

It is preferable that the state of the target component be a concentration of the target component.

It is preferable that the target component be a protein.

It is preferable that the protein be an antibody.

It is preferable that the electromagnetic waves be Raman scattering light.

It is preferable that the state prediction model be a machine learning model that has been trained using the standard spectral data as supervised training data.

According to the present disclosure, there is provided an operation method of an information processing apparatus that predicts a state of a target component in a suspension in which biological molecules are dispersed as components in a liquid, based on spectral data obtained by measuring electromagnetic waves emitted from the suspension, the operation method comprising: using a state prediction model calibrated with standard spectral data obtained from a standard suspension in a standard measurement environment; acquiring target spectral data obtained from a target suspension in which the state of the target component is unknown, in a target measurement environment different from the standard measurement environment; acquiring a correction coefficient derived by comparing the standard spectral data with reference spectral data that corresponds to the target spectral data and that is obtained from the standard suspension in the target measurement environment; correcting the target spectral data with the correction coefficient to generate corrected target spectral data; and applying the corrected target spectral data to the state prediction model so that the state prediction model predicts the state of the target component.

According to the present disclosure, there is provided an operation program of an information processing apparatus that predicts a state of a target component in a suspension in which biological molecules are dispersed as components in a liquid, based on spectral data obtained by measuring electromagnetic waves emitted from the suspension, the operation program causing a computer to execute a process comprising: using a state prediction model calibrated with standard spectral data obtained from a standard suspension in a standard measurement environment; acquiring target spectral data obtained from a target suspension in which the state of the target component is unknown, in a target measurement environment different from the standard measurement environment; acquiring a correction coefficient derived by comparing the standard spectral data with reference spectral data that corresponds to the target spectral data and that is obtained from the standard suspension in the target measurement environment; correcting the target spectral data with the correction coefficient to generate corrected target spectral data; and applying the corrected target spectral data to the state prediction model so that the state prediction model predicts the state of the target component.

According to the technology of the present disclosure, it is possible to provide an information processing apparatus, an operation method of an information processing apparatus, and an operation program of an information processing apparatus that are capable of obtaining a highly reliable prediction result for a state of a target component in a suspension even in a case where a measurement environment of electromagnetic waves is changed.

### BRIEF DESCRIPTION OF THE DRAWINGS

FIG. 1 is a diagram showing a concentration section, a measurement system, and an information processing apparatus.
FIG. 2 is a diagram showing excitation light and Raman scattering light.
FIG. 3 is a diagram showing Raman spectral data.
FIG. 4 is a cross-sectional view of a flow cell.
FIG. 5 is a block diagram of a computer constituting the information processing apparatus.
FIG. 6 is a block diagram showing a processing unit of a CPU of the information processing apparatus.
FIG. 7 is a diagram showing correction coefficient information.
FIG. 8 is a diagram showing a state of measuring standard Raman spectral data and reference Raman spectral data.
FIG. 9 is a table showing contents of standard suspensions.
FIG. 10 is a diagram showing correction coefficient derivation processing.
FIG. 11 is a diagram showing a used flow cell selection screen.
FIG. 12 is a diagram showing a state of reading out and acquiring a correction coefficient corresponding to a designation of a type of a used flow cell from a storage.
FIG. 13 is a diagram showing processing executed by a correction unit.
FIG. 14 is a diagram showing processing executed by a prediction unit.
FIG. 15 is a diagram showing a neural network constituting an antibody concentration prediction model.
FIG. 16 is a diagram showing a process in a learning phase of the antibody concentration prediction model.
FIG. 17 is a diagram showing a prediction result display screen.
FIG. 18 is a flowchart showing a processing procedure of the information processing apparatus.
FIG. 19 is a diagram showing another example of the correction coefficient information.
FIG. 20 is a diagram showing still another example of the correction coefficient information.
FIG. 21 is a diagram showing a second embodiment in which the antibody concentration prediction model is trained using corrected target Raman spectral data.
FIG. 22 is a diagram showing a predicted value and an actual measurement value of an antibody concentration, as well as an average error thereof, in Examples.
FIG. 23 is a table showing contents of Comparative Examples.
FIG. 24 is a diagram showing a predicted value and an actual measurement value of an antibody concentration, as well as an average error thereof, in Comparative Example 1.
FIG. 25 is a diagram showing a predicted value and an actual measurement value of an antibody concentration, as well as an average error thereof, in Comparative Example 2.
FIG. 26 is a diagram showing a predicted value and an actual measurement value of an antibody concentration, as well as an average error thereof, in Comparative Example 3.

### DESCRIPTION OF THE PREFERRED EMBODIMENTS

### [First Embodiment]

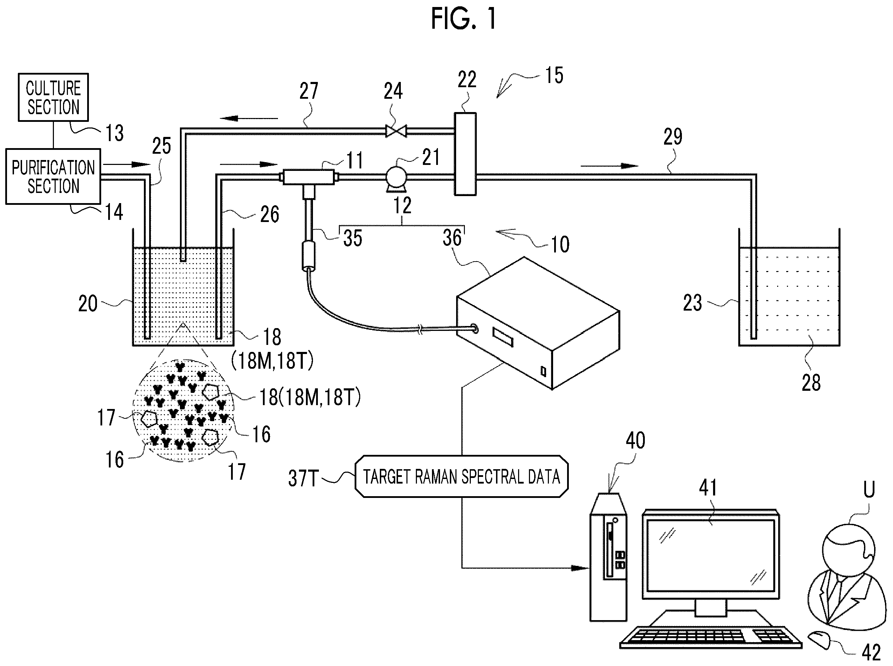

As shown in FIG. 1 as an example, a measurement system 10 comprises a flow cell 11 and a Raman spectrometer 12. The measurement system 10 is incorporated into, for example, a concentration section 15 following a culture section 13 and a purification section 14 in a biopharmaceutical drug substance manufacturing system.

The culture section 13 has a culture tank and a cell removal filter. A cell culture solution (medium) is stored in the culture tank. Antibody-producing cells are seeded in the cell culture solution, and the antibody-producing cells are cultured in the cell culture solution. The culture method may be either perfusion culture or fed-batch culture. The antibody-producing cells are, for example, cells established by incorporating antibody genes into host cells such as Chinese hamster ovary cells (CHO cells). The antibody-producing cells produce immunoglobulins, that is, antibodies 16 during the culture process. Therefore, not only the antibody-producing cells but also the antibodies 16 are present in the cell culture solution. The antibody 16 is, for example, a monoclonal antibody and serves as an active ingredient of a biopharmaceutical. The antibody 16 is an example of a "component", a "target component", and a "protein" according to the technology of the present disclosure.

The cell removal filter uses, for example, a tangential flow filtration (TFF) method or an alternating tangential flow filtration (ATF) method to capture the antibody-producing cells in the cell culture solution with a filter membrane and remove the antibody-producing cells from the cell culture solution. In addition, the cell removal filter allows the antibody 16 to pass through. Therefore, the cell culture solution flowing from the culture section 13 to the purification section 14 mainly contains the antibody 16. The cell culture solution from which the antibody-producing cells have been removed using the cell removal filter is called a culture supernatant. The culture supernatant includes a cell-derived protein/cell-derived deoxyribonucleic acid (DNA), an aggregate 17 of the antibody 16, a virus, and the like in addition to the antibody 16. These cell-derived protein/cell-derived DNA, the aggregate 17 of the antibody 16, the virus, and the like are also examples of the "component" according to the technology of the present disclosure.

The purification section 14 sequentially performs component separation processing using a plurality of chromatography devices on the culture supernatant from the culture section 13, thereby gradually removing impurities and viruses and gradually increasing the purity of the antibody 16 in the culture supernatant. Examples of the plurality of chromatography devices include an Immunoaffinity chromatography device, a size exclusion chromatography device, a cation chromatography device, an anion chromatography device, and a hydrophobic interaction chromatography device.

In addition, the purification section 14 performs a process of inactivating viruses in the culture supernatant and a process of removing viruses in the culture supernatant using a filter. A purified solution 18 obtained through such various processes is sent from the purification section 14 to the concentration section 15. A single-pass tangential flow filtration (SPTFF) filter may be provided upstream of each chromatography device.

The concentration section 15 is provided with a primary tank 20, a pump 21, a concentration filter 22, a secondary tank 23, a pressure control valve 24, and the like. A flow-in path 25, a flow-out path 26, and a return flow path 27 are connected to the primary tank 20. The purified solution 18 from the purification section 14 flows into the primary tank 20 through the flow-in path 25. The primary tank 20 stores the purified solution 18.

The pump 21 is provided in the flow-out path 26. By driving the pump 21, the purified solution 18 stored in the primary tank 20 is sent to the flow-out path 26 and is introduced into the concentration filter 22 through the flow-out path 26. The concentration filter 22 subjects the introduced purified solution 18 to concentration/filtration processing, for example, by ultrafiltration (UF) and diafiltration (DF).

Through the concentration/filtration processing by the concentration filter 22, the purified solution 18 is separated into a concentrated solution containing the antibody 16 with a further increased purity, and a waste liquid 28 including water, a solvent, or the like. The concentrated solution is returned to the primary tank 20 through the return flow path 27. Therefore, the liquid stored in the primary tank 20 is a mixed solution 18M of the purified solution 18 from the purification section 14 and the concentrated solution from the concentration filter 22. The purified solution 18 and the mixed solution 18M are examples of a "suspension" and a "target suspension" according to the technology of the present disclosure. Hereinafter, the purified solution 18 and the mixed solution 18M will be collectively referred to as a target suspension 18T, unless there is a particular need to distinguish between them.

The waste liquid 28 is introduced into the secondary tank 23 through a waste liquid path 29. The secondary tank 23 stores the waste liquid 28.

The pressure control valve 24 is provided in the return flow path 27. By controlling opening and closing of the pressure control valve 24, a liquid pressure of the concentrated solution flowing through the return flow path 27 is held constant. Although not shown, a pressure gauge is provided on a downstream side of the pump 21 in the flow-out path 26, on an upstream side of the pressure control valve 24 in the return flow path 27, and in the waste liquid path 29. In addition, the primary tank 20 and the secondary tank 23 are placed on a weighing scale. The operation of the pump 21 and the pressure control valve 24 is controlled in accordance with the pressure of each liquid measured by the pressure gauge and the weight of the primary tank 20 and the secondary tank 23 measured by the weighing scale.

The flow cell 11 is connected to an upstream side of the pump 21 in the flow-out path 26. The target suspension 18T from the primary tank 20 flows through the flow cell 11 at a preset flow rate.

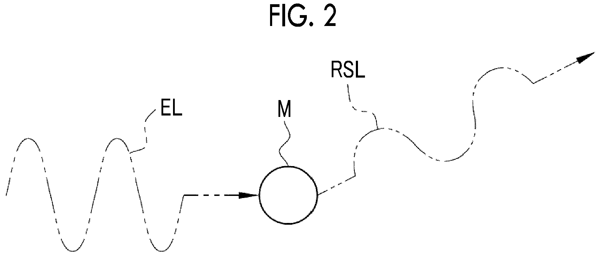

As shown in FIG. 2 as an example, the Raman spectrometer 12 is a device that evaluates a substance M by using the characteristics of Raman scattering light RSL. In a case where the substance M is irradiated with excitation light EL, the excitation light EL interacts with the substance M to generate Raman scattering light RSL having a wavelength different from that of the excitation light EL. A wavelength difference between the excitation light EL and the Raman scattering light RSL corresponds to the energy of the molecular vibration of the substance M. For this reason, it is possible to obtain the Raman scattering light RSL having different wave numbers between the substances M having different molecular structures. The Raman scattering light RSL is an example of an "electromagnetic wave" according to the technology of the present disclosure. Out of the Stokes line and the anti-Stokes line, it is preferable to use the Stokes line as the Raman scattering light RSL.

The Raman spectrometer 12 includes a sensor unit 35 and an analyzer 36. The sensor unit 35 is connected to the flow cell 11. The sensor unit 35 emits the excitation light EL from its tip. The excitation light EL is emitted to the target suspension 18T flowing inside the flow cell 11. The Raman scattering light RSL is generated by the interaction between the excitation light EL and the component such as the antibody 16 in the target suspension 18T. The sensor unit 35 receives the Raman scattering light RSL, and outputs the received Raman scattering light RSL to the analyzer 36.

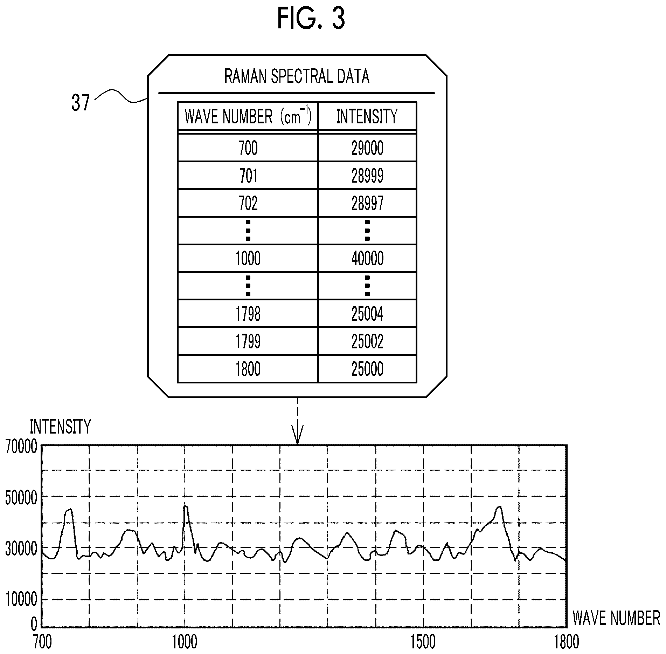

The analyzer 36 decomposes the Raman scattering light RSL for each wave number and derives an intensity value of the Raman scattering light RSL for each wave number, thereby generating Raman spectral data 37 representing a spectrum of the Raman scattering light RSL, that is, a Raman spectrum, as shown in FIG. 3 as an example. The Raman spectral data 37 is data in which the intensity value of the Raman scattering light RSL is registered for each wave number. In this example, the Raman spectral data 37 is data in which intensity values of the Raman scattering light RSL in a range of wave numbers of 700 cm⁻¹ to 1800 cm⁻¹ are derived in increments of 1 cm⁻¹. In addition, a graph shown below the Raman spectral data 37 in FIG. 3 is a graph in which the intensity value of the Raman spectral data 37 is plotted for each wave number and the plotted points are connected by a line. The Raman spectral data 37 is an example of "spectral data" according to the technology of the present disclosure.

As described above, the measurement system 10 causes the target suspension 18T to flow through the flow cell 11. The Raman scattering light RSL of the component such as the antibody 16 in the target suspension 18T is measured by irradiating the target suspension 18T flowing through the flow cell 11 with the excitation light EL through the sensor unit 35, and the Raman spectral data 37 is obtained. In addition, the measurement environment of the Raman scattering light RSL using the flow cell 11 shown in FIG. 1 is an example of a "target measurement environment" according to the technology of the present disclosure.

An information processing apparatus 40 is connected to the Raman spectrometer 12 through a computer network such as a local area network (LAN). The Raman spectrometer 12 transmits the measured Raman spectral data 37 to the information processing apparatus 40. The Raman spectral data 37 transmitted from the Raman spectrometer 12 to the information processing apparatus 40 is data for predicting a concentration of the antibody 16. The concentration of the antibody 16 is an example of a "state of the target component" according to the technology of the present disclosure. In the following description, the Raman spectral data 37 transmitted from the Raman spectrometer 12 to the information processing apparatus 40 will be referred to as target Raman spectral data 37T. The target Raman spectral data 37T is an example of "target spectral data" according to the technology of the present disclosure.

The information processing apparatus 40 is, for example, a desktop personal computer, and comprises a display 41 that displays various screens and an input device 42, such as a keyboard, a mouse, a touch panel, and/or a microphone for voice input. The information processing apparatus 40 is installed in, for example, a pharmaceutical company that develops biopharmaceuticals, or in an organization that undertakes development work of biopharmaceuticals from a pharmaceutical company, that is, a contract research organization (CRO). The information processing apparatus 40 is operated by a user U who is involved in the development of the biopharmaceuticals in the pharmaceutical company or the contract research organization (hereinafter, collectively referred to as a pharmaceutical facility).

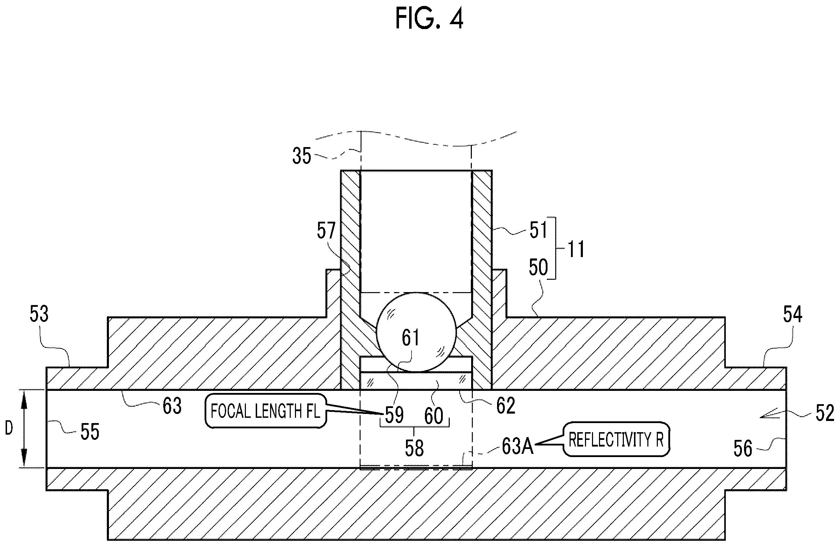

As shown in FIG. 4 as an example, the flow cell 11 is configured with a main body 50 and a sensor unit connector 51. The main body 50 is a cylindrical member having a linear flow path 52 with a circular cross section at a center thereof. The main body 50 is made of a metal, for example, Hastelloy. Alternatively, the main body 50 may be made of a resin, for example, a polyolefin-based resin.

A first connecting portion 53 and a second connecting portion 54 each having a cylindrical boss shape are provided at centers of both end surfaces of the main body 50. The first connecting portion 53 has an inlet 55 of the flow path 52, and the second connecting portion 54 has an outlet 56 of the flow path 52. The first connecting portion 53 and the second connecting portion 54 are, for example, straight threads or tapered threads. A sterile connector provided in the flow-out path 26 is liquid-tightly attached to the first connecting portion 53 and the second connecting portion 54.

An attachment hole 57 for attachably and detachably attaching the sensor unit connector 51 to the main body 50 is formed at a center of a peripheral surface of the main body 50. The attachment hole 57 has a thread formed on its inner peripheral surface. The attachment hole 57 passes through the flow path 52. A tip part of the sensor unit connector 51 is formed with a thread that screws into the thread on the inner peripheral surface of the attachment hole 57. By screwing the threads to each other to attach the sensor unit connector 51 to the attachment hole 57, the tip part of the sensor unit connector 51 is housed within the attachment hole 57. The tip of the sensor unit 35 is attachably and detachably connected to the sensor unit connector 51.

The sensor unit connector 51 is a cylindrical member with an optical system 58 disposed at the tip. The optical system 58 is configured with a ball lens 59 and a transparent plate 60. Optical axes of the ball lens 59 and the transparent plate 60 are aligned. The ball lens 59 is literally a spherical lens, and is made of, for example, sapphire glass or quartz glass. The ball lens 59 focuses the excitation light EL from the sensor unit 35 and introduces the Raman scattering light RSL, which is generated by the interaction between the excitation light EL and the antibody 16 or the like in the target suspension 18T, into the sensor unit 35.

The transparent plate 60 is also made of, for example, sapphire glass or quartz glass, as with the ball lens 59. The transparent plate 60 is a disk in which a first surface 61 on the ball lens 59 side and a second surface 62 on the flow path 52 side are parallel to each other. The second surface 62 is in contact with the target suspension 18T flowing through the flow path 52. The second surface 62 is an example of an "incident surface" according to the technology of the present disclosure. Here, the term "parallel" refers to parallel in a meaning including an error that is generally allowed in the technical field to which the technology of the present disclosure belongs, and an error to such an extent not contrary to the spirit and scope of the technology of the present disclosure, in addition to completely parallel.

A focusing position of the ball lens 59 is located, for example, on the second surface 62 of the transparent plate 60. The focusing position of the ball lens 59 is determined by a diameter, a refractive index, a focal length FL, and the like of the ball lens 59. The focusing position in this case has a dot shape. By setting the focusing position of the ball lens 59 to a position on the second surface 62 of the transparent plate 60, it is possible to reduce the risk of the excitation light EL being attenuated by the target suspension 18T. As a result, an S/N ratio of the Raman spectral data 37 can be maintained at a high level.

A wall surface 63 forming the flow path 52 is, for example, a smooth surface with no irregularities of 1 mm or more. A wall surface 63A facing the second surface 62 of the transparent plate 60 has a reflectivity R. In addition, a distance D between the second surface 62 and the wall surface 63A is substantially the same as a diameter of the flow path 52. The diameter of the flow path 52 is set in accordance with a flow rate and a viscosity of the target suspension 18T flowing through the flow path 52, a size of the concentration filter 22, and the like. The diameter of the flow path 52 is in a range in which a Reynolds number Re in the flow path 52 is 2300 or more (Re ≥ 2300), for example, under a condition in which the flow rate of the target suspension 18T flowing through the flow path 52 is 200 cc/min or more and the viscosity of the target suspension 18T is 0.001 Pa·s, which is the same as that of water. In a case where the Reynolds number Re is 2300 or more, turbulence occurs in the target suspension 18T flowing through the flow path 52. Accordingly, a component deviation in the target suspension 18T is reduced, so that the measurement stability of the Raman spectral data 37 can be improved.

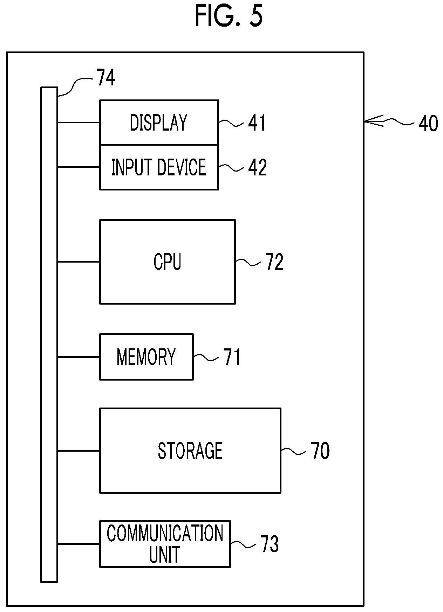

As shown in FIG. 5 as an example, the computer constituting the information processing apparatus 40 comprises a storage 70, a memory 71, a central processing unit (CPU) 72, and a communication unit 73, in addition to the display 41 and the input device 42 described above. These are interconnected via a busline 74.

The storage 70 is a hard disk drive that is incorporated in the computer constituting the information processing apparatus 40 or connected to the computer through a cable or a network. Alternatively, the storage 70 is a disk array configured with a plurality of hard disk drives. The storage 70 stores a control program such as an operating system, various application programs, various data associated with these programs, and the like. The storage 70 is an example of a "storage unit" according to the technology of the present disclosure. A solid state drive may be used instead of the hard disk drive.

The memory 71 is a work memory for the CPU 72 to execute processing. The CPU 72 loads the program stored in the storage 70 into the memory 71 and executes processing according to the program. Thus, the CPU 72 collectively controls the respective units of the computer. The CPU 72 is an example of a "processor" according to the technology of the present disclosure. The memory 71 may be built in the CPU 72. The communication unit 73 performs transmission control of various information with an external device such as the Raman spectrometer 12.

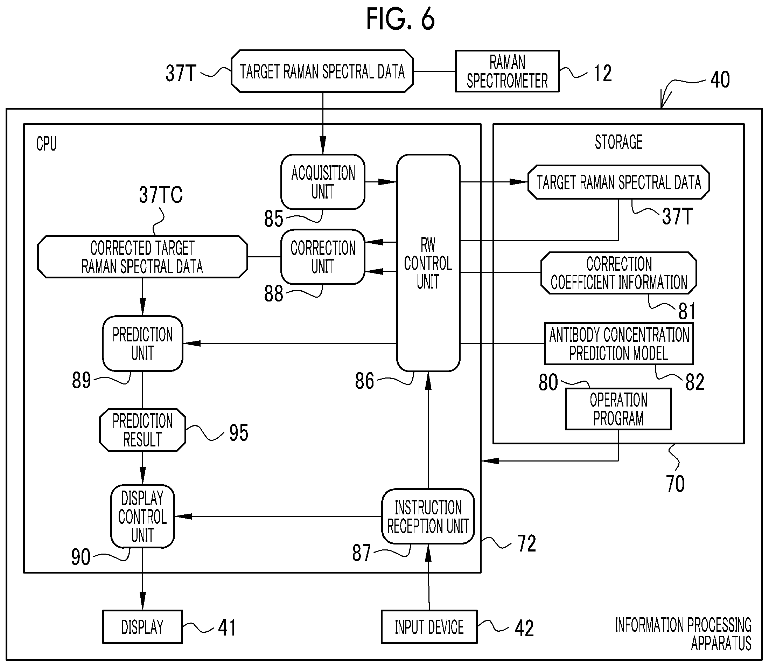

As shown in FIG. 6 as an example, an operation program 80 is stored in the storage 70. The operation program 80 is an application program for causing the computer to function as the information processing apparatus 40. The storage 70 also stores correction coefficient information 81 and an antibody concentration prediction model 82, in addition to the operation program 80. The antibody concentration prediction model 82 is an example of a "state prediction model" according to the technology of the present disclosure.

In a case where the operation program 80 is activated, the CPU 72 of the computer constituting the information processing apparatus 40 functions as an acquisition unit 85, a read/write (hereinafter, abbreviated as RW) control unit 86, an instruction reception unit 87, a correction unit 88, a prediction unit 89, and a display control unit 90 in cooperation with the memory 71 and the like.

The acquisition unit 85 acquires the target Raman spectral data 37T from the Raman spectrometer 12. The acquisition unit 85 outputs the target Raman spectral data 37T to the RW control unit 86.

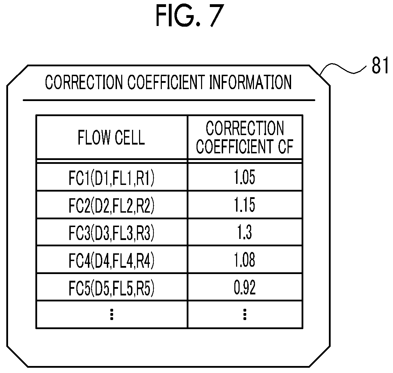

The RW control unit 86 controls the storage of various data in the storage 70 and the read-out of various data stored in the storage 70. The RW control unit 86 stores the target Raman spectral data 37T from the acquisition unit 85 in the storage 70. In addition, the RW control unit 86 reads out the target Raman spectral data 37T and a correction coefficient CF (see FIG. 7) registered in the correction coefficient information 81 from the storage 70, and outputs the read-out target Raman spectral data 37T and the read-out correction coefficient CF to the correction unit 88. Further, the RW control unit 86 reads out the antibody concentration prediction model 82 from the storage 70, and outputs the read-out antibody concentration prediction model 82 to the prediction unit 89.

The instruction reception unit 87 receives various operation instructions input by the user U via the input device 42.

The correction unit 88 corrects the target Raman spectral data 37T with the correction coefficient CF to generate corrected target Raman spectral data 37TC. The correction unit 88 outputs the corrected target Raman spectral data 37TC to the prediction unit 89.

The prediction unit 89 applies the corrected target Raman spectral data 37TC to the antibody concentration prediction model 82 so that the antibody concentration prediction model 82 predicts the concentration of the antibody 16 in the target suspension 18T, and a prediction result 95 is output from the antibody concentration prediction model 82. The prediction unit 89 outputs the prediction result 95 to the display control unit 90.

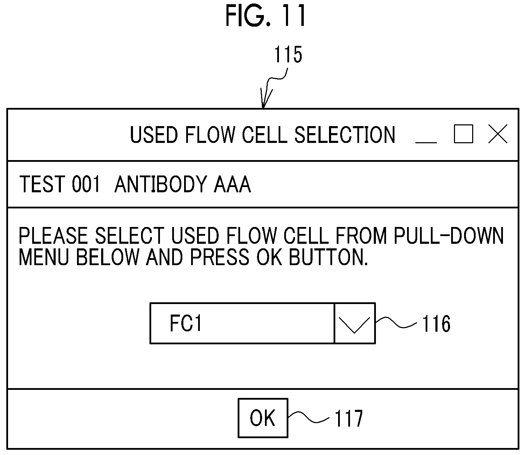

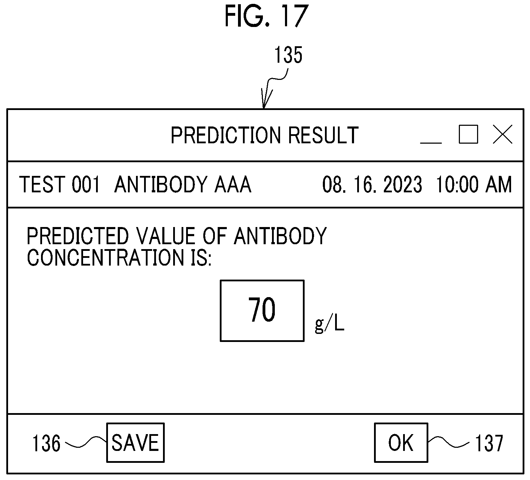

The display control unit 90 controls the display of the various screens on the display 41. For example, the display control unit 90 performs control of displaying, on the display 41, a used flow cell selection screen 115 (see FIG. 11) for allowing the user U to select the flow cell 11 used, a prediction result display screen 135 (see FIG. 17) for displaying the prediction result 95, and the like.

As shown in FIG. 7 as an example, the correction coefficient information 81 is information in which correction coefficients CF of a plurality of types of flow cells 11 (product names FC1, FC2, FC3, ...) that can be used in a biopharmaceutical manufacturing system are comprehensively registered. The plurality of types of flow cells 11 differ in at least one of the distance D between the second surface 62 of the transparent plate 60 and the wall surface 63A of the flow path 52 facing the second surface 62, the reflectivity R of the wall surface 63A, or the focal length FL of the ball lens 59. In addition, the correction coefficient information 81 is not limited to an aspect in which the flow cell 11 is used, and the correction coefficient CF for an aspect in which the Raman scattering light RSL is measured by directly immersing the sensor unit 35 in a tank (chamber) in which the target suspension 18T is stored without using the flow cell 11 can also be registered.

In the biopharmaceutical manufacturing system, for example, the plurality of types of flow cells 11 that are different from each other are selectively used in accordance with the scale of the equipment. Specifically, conditions are set using relatively small-scale equipment, and, in a case of shifting to relatively large-scale equipment after the condition setting is completed, the flow cell is changed in accordance with the large-scale equipment. A plurality of measurement environments in which the plurality of types of flow cells 11 that are different from each other are selectively used are examples of "a plurality of target measurement environments" according to the technology of the present disclosure.

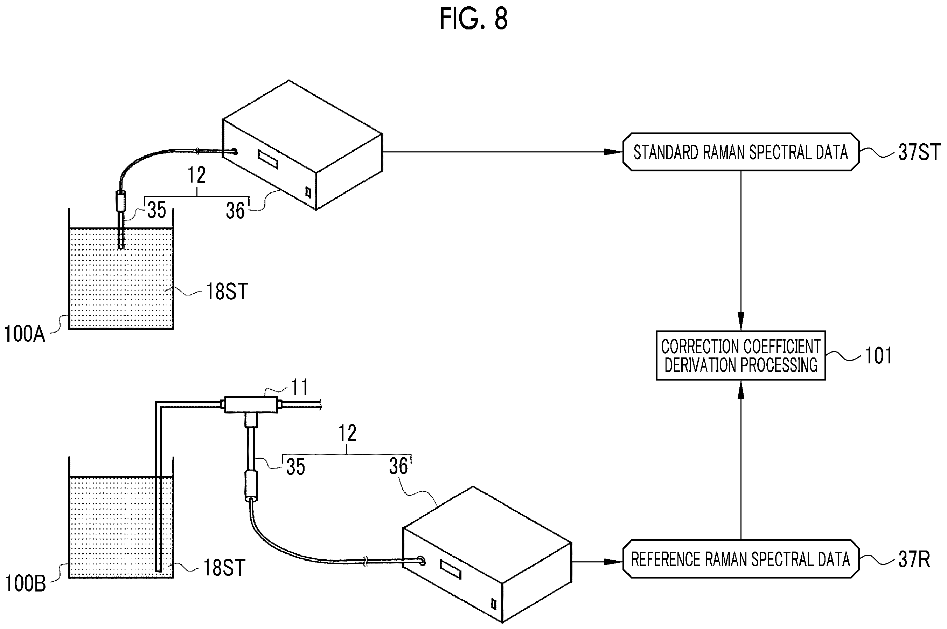

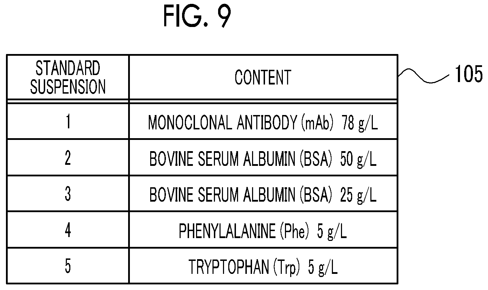

The correction coefficient CF is derived as follows. First, as shown in FIG. 8 as an example, a standard suspension 18ST is prepared in two tanks 100A and 100B. As shown in a table 105 of FIG. 9 as an example, there are five types of the standard suspension 18ST. Specifically, the standard suspension 18ST includes a standard suspension 1 that is a 78 g/L solution of a monoclonal antibody (mAb), a standard suspension 2 that is a 50 g/L solution of bovine serum albumin (BSA), and a standard suspension 3 that is a 25 g/L solution of bovine serum albumin. In addition, the standard suspension 18ST includes a standard suspension 4 that is a 5 g/L solution of phenylalanine (Phe), and a standard suspension 5 that is a 5 g/L solution of tryptophan (Trp).

The sensor unit 35 of the Raman spectrometer 12 is directly immersed in the standard suspension 18ST in the tank 100A, and the Raman scattering light RSL from the standard suspension 18ST is measured, thereby obtaining standard Raman spectral data 37ST. The measurement environment of the Raman scattering light RSL without using the flow cell 11 is an example of a "standard measurement environment" according to the technology of the present disclosure.

On the other hand, the standard suspension 18ST in the tank 100B is caused to flow through the flow cell 11, the sensor unit 35 is connected to the flow cell 11, and the Raman scattering light RSL from the standard suspension 18ST flowing through the flow cell 11 is measured, thereby obtaining reference Raman spectral data 37R. The reference Raman spectral data 37R is Raman spectral data corresponding to the target Raman spectral data 37T. The measurement environment of the reference Raman spectral data 37R uses the flow cell 11, and is therefore an example of a "target measurement environment" according to the technology of the present disclosure, similar to the measurement environment shown in FIG. 1. That is, the standard measurement environment and the target measurement environment in this example differ in whether the flow cell 11 is used. In addition, the standard measurement environment and the target measurement environment may differ in the type of the flow cell 11. That is, in the standard measurement environment as well, the flow cell 11 having preset specifications may be used.

The standard Raman spectral data 37ST and the reference Raman spectral data 37R obtained in this way are subjected to correction coefficient derivation processing 101.

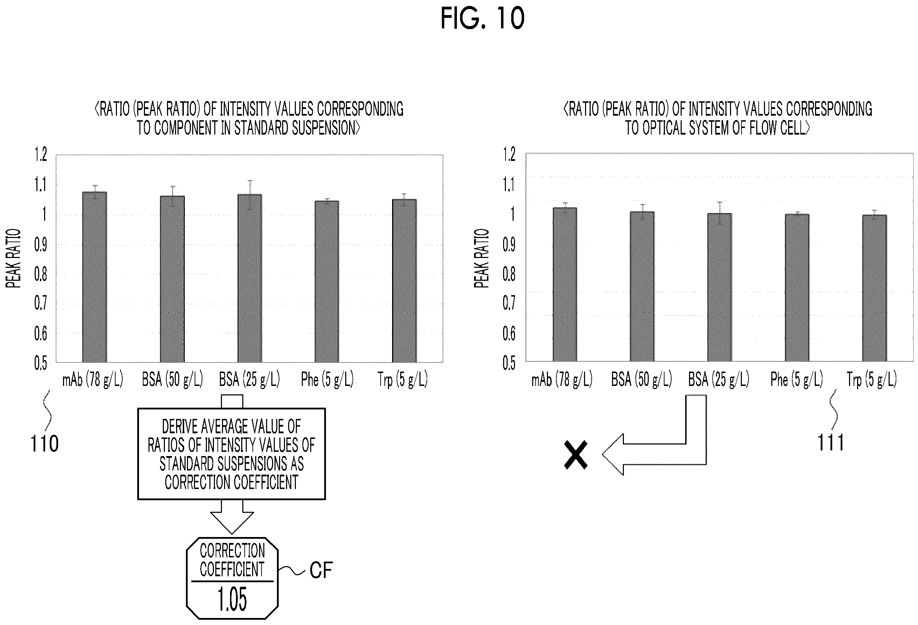

As shown in a graph 110 of FIG. 10 as an example, the correction coefficient derivation processing 101 starts with calculating a ratio (peak ratio) of an intensity value of the standard Raman spectral data 37ST corresponding to the component in the standard suspension 18ST to an intensity value of the reference Raman spectral data 37R corresponding to the component in the standard suspension 18ST for each of the suspension suspensions 1 to 5. The component in the standard suspension 18ST is the monoclonal antibody in a case of the standard suspension 1, and is the phenylalanine in a case of the standard suspension 4. For example, in a case where the intensity value of the standard Raman spectral data 37ST corresponding to the component in the standard suspension 18ST is 100 and the intensity value of the reference Raman spectral data 37R corresponding to the component in the standard suspension 18ST is 95, the ratio thereof is 100/95 ≈ 1.05. After calculating the ratio of the intensity values for each of the standard suspensions 1 to 5 in this way, an average value of the ratios of the intensity values is calculated. The average value is set as the correction coefficient CF.

Here, in a case where the flow cell 11 is used, not only the peak corresponding to the component in the target suspension 18T or the standard suspension 18ST, but also a peak derived from the optical system 58 of the flow cell 11, such as a peak derived from sapphire glass that is a material of the ball lens 59, is observed in the Raman spectral data 37. The peak derived from the optical system 58 is not observed in the standard measurement environment of this example in which the flow cell 11 is not used, or the position and/or the intensity value of the peak is significantly different. Therefore, as shown in a graph 111, a ratio of an intensity value of the standard Raman spectral data 37ST corresponding to the optical system 58 to an intensity value of the reference Raman spectral data 37R corresponding to the optical system 58 is a value that deviates from the ratio of the intensity value of the standard Raman spectral data 37ST corresponding to the component in the standard suspension 18ST to the intensity value of the reference Raman spectral data 37R corresponding to the component in the standard suspension 18ST. Therefore, in this example, as indicated by the mark "×", the correction coefficient CF is derived by excluding the ratio of the intensity values corresponding to the optical system 58.

In a case where the instruction reception unit 87 receives an instruction from the user U to predict the concentration of the antibody 16 with the target Raman spectral data 37T, the display control unit 90 performs control of displaying, on the display 41, the used flow cell selection screen 115 shown in FIG. 11 as an example. The used flow cell selection screen 115 is provided with a pull-down menu 116 for selectively selecting the flow cell 11 used in a case of obtaining the target Raman spectral data 37T. In the pull-down menu 116, characters that uniquely identify the flow cell 11, such as a product name and a model number (here, the product name is exemplified) of the flow cell 11, are listed in a selectable manner. The user U selects the used flow cell 11 from the pull-down menu 116 and selects an OK button 117.

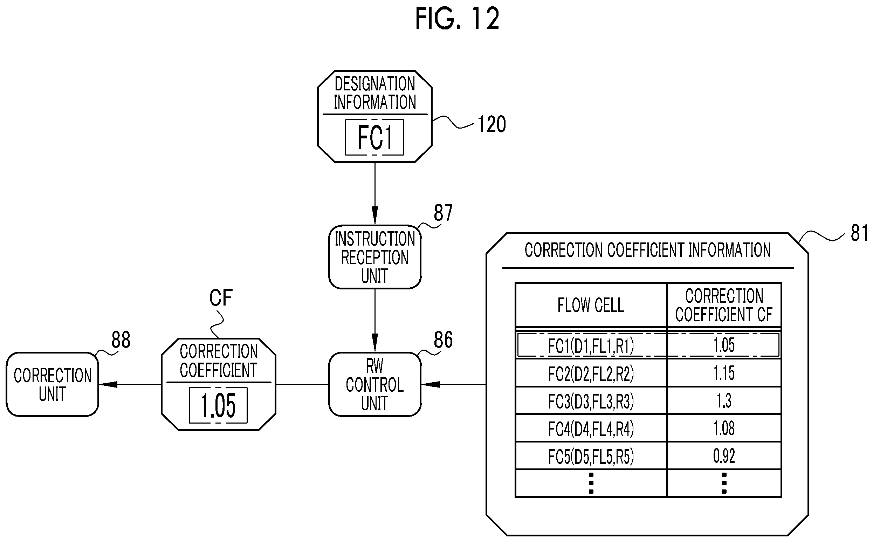

On the used flow cell selection screen 115, in a case where the user U selects the used flow cell 11 from the pull-down menu 116 and selects the OK button 117, as shown in FIG. 12 as an example, the instruction reception unit 87 receives designation information 120 of the used flow cell 11. The designation information 120 includes the product name, the model number, and the like (here, the product name is exemplified) of the flow cell 11. The instruction reception unit 87 outputs the designation information 120 to the RW control unit 86.

The RW control unit 86 acquires the correction coefficient CF corresponding to the flow cell 11 designated by the designation information 120 by reading out the correction coefficient CF from the correction coefficient information 81 of the storage 70. The RW control unit 86 outputs the read-out correction coefficient CF to the correction unit 88. The correction unit 88 corrects the target Raman spectral data 37T with the correction coefficient CF from the RW control unit 86. In FIG. 12, an example is shown in which the flow cell 11 having a product name "FC1" is selected as the used flow cell 11, and "1.05" is read out from the correction coefficient information 81 as the correction coefficient CF and is output to the correction unit 88.

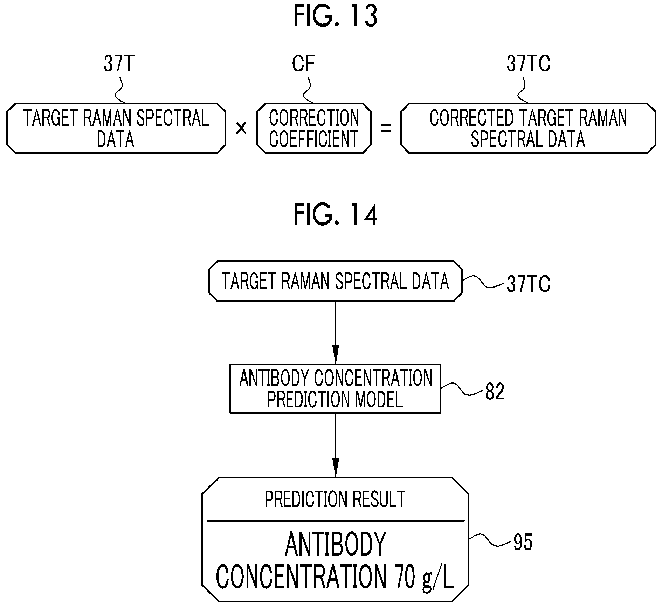

As shown in FIG. 13 as an example, the correction unit 88 converts the target Raman spectral data 37T into the corrected target Raman spectral data 37TC by uniformly multiplying all the intensity values of the target Raman spectral data 37T by the correction coefficient CF.

As shown in FIG. 14 as an example, the prediction unit 89 inputs the corrected target Raman spectral data 37TC to the antibody concentration prediction model 82, and outputs the prediction result 95 from the antibody concentration prediction model 82. The prediction result 95 includes a predicted value of the concentration of the antibody 16 in the target suspension 18T.

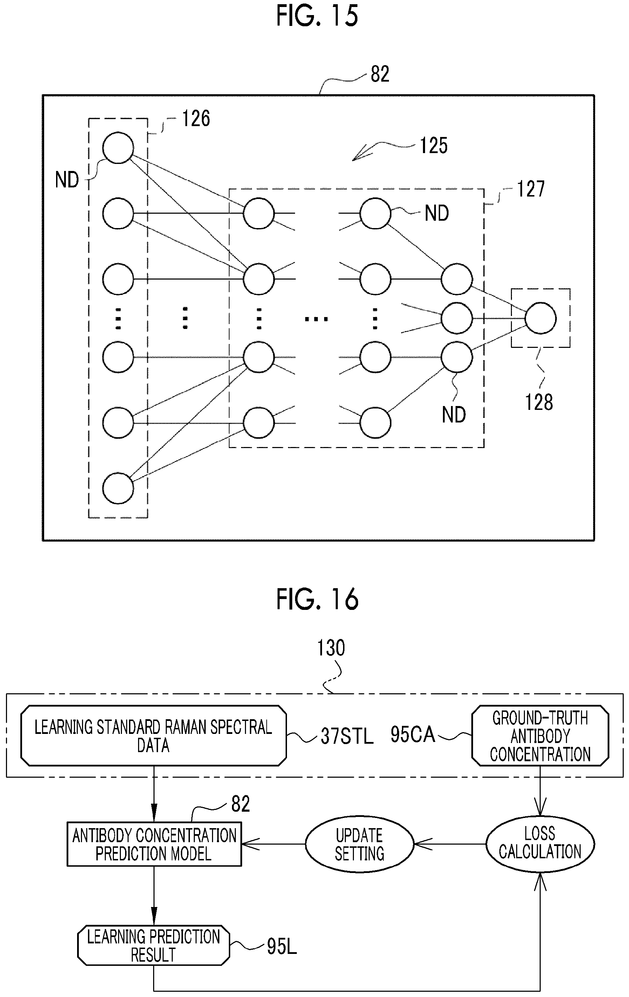

As shown in FIG. 15 as an example, the antibody concentration prediction model 82 is constructed by a neural network 125. Therefore, the antibody concentration prediction model 82 is also an example of a "machine learning model" according to the technology of the present disclosure. As is well known, the neural network 125 has an input layer 126, an interlayer (also referred to as a hidden layer) 127, and an output layer 128. The input layer 126, the interlayer 127, and the output layer 128 each have a plurality of nodes ND. A coefficient indicating the connection strength between the respective nodes ND is set between the node ND of the input layer 126 and the node ND of the interlayer 127, between the nodes ND of the interlayer 127, and between the node ND of the interlayer 127 and the node ND of the output layer 128. A suitable activation function such as a linear function or a rectified linear unit (ReLU) function is set for the node ND of the output layer 128.

The intensity value of each wave number of the corrected target Raman spectral data 37TC is input to each node ND of the input layer 126. In addition, the predicted value of the concentration of the antibody 16 is output from the node ND of the output layer 128. In addition, the antibody concentration prediction model 82 is not limited to the illustrated neural network 125, and may be another machine learning model such as a decision tree, a gradient boosting decision tree, a random forest, a support vector machine, and a naive Bayes model.

As shown in FIG. 16 as an example, in a learning phase of the antibody concentration prediction model 82, supervised training data (also referred to as learning data or training data) 130 is used. The supervised training data 130 is a set of learning standard Raman spectral data 37STL and a ground-truth antibody concentration 95CA. The learning standard Raman spectral data 37STL is obtained by measuring the Raman scattering light RSL emitted from the standard suspension 18ST including at least the antibody 16 in the standard measurement environment shown in FIG. 8. The ground-truth antibody concentration 95CA is a concentration of the antibody 16 in the standard suspension 18ST measured using a mass spectrometry function of a high performance liquid chromatography (hereinafter, referred to as HPLC) device into which the standard suspension 18ST that is the basis of the learning standard Raman spectral data 37STL is introduced.

In the learning phase, the learning standard Raman spectral data 37STL is input to the antibody concentration prediction model 82, and a learning prediction result 95L is output from the antibody concentration prediction model 82. Next, a loss calculation of the antibody concentration prediction model 82 using a loss function is performed based on a comparison result between the learning prediction result 95L and the ground-truth antibody concentration 95CA. Then, coefficients (coefficients of the nodes ND) of the antibody concentration prediction model 82 are updated according to the result of the loss calculation, and the antibody concentration prediction model 82 is updated according to the update setting.

In the learning phase, the series of processing of inputting the learning standard Raman spectral data 37STL to the antibody concentration prediction model 82, outputting the learning prediction result 95L from the antibody concentration prediction model 82, performing the loss calculation, performing the update setting, and updating the antibody concentration prediction model 82 is repeatedly performed while changing the supervised training data 130. The repetition of the series of processing is ended in a case where the prediction accuracy of the learning prediction result 95L for the ground-truth antibody concentration 95CA has reached a predetermined set level. The antibody concentration prediction model 82 of which the prediction accuracy has reached the set level in this way is stored in the storage 70 and is used by the prediction unit 89. The repetition of the series of processing is an example of "calibration" according to the technology of the present disclosure. The learning may be ended in a case where the above-described series of processing is repeated a set number of times, regardless of the prediction accuracy of the learning prediction result 95L for the ground-truth antibody concentration 95CA.

In response to the prediction result 95 from the prediction unit 89, the display control unit 90 performs control of displaying, on the display 41, the prediction result display screen 135 shown in FIG. 17 as an example. The prediction result display screen 135 displays the predicted value of the concentration of the antibody 16 of the prediction result 95. In addition, a save button 136 and an OK button 137 are provided at a lower part of the prediction result display screen 135. In a case where the save button 136 is selected, the predicted value is stored in the storage 70 in association with the target Raman spectral data 37T. In a case where the OK button 137 is selected, the display of the prediction result display screen 135 disappears. In addition, for example, the concentration of the antibody 16 may be predicted by the antibody concentration prediction model 82 at a plurality of time points at regular intervals, the predicted values of the concentration of the antibody 16 at the plurality of time points may be obtained, the predicted values of the concentration of the antibody 16 at the plurality of time points may be plotted on a graph with time on a horizontal axis, and a time-series change in the predicted values of the concentration of the antibody 16 may be displayed on the prediction result display screen 135.

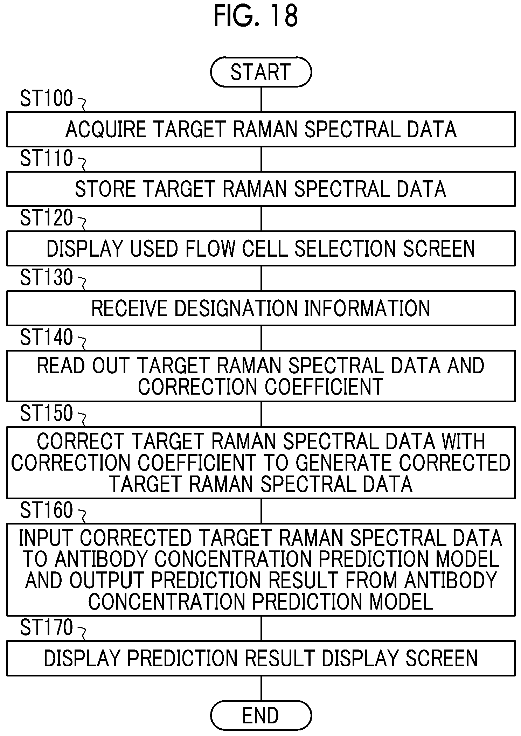

Next, an operation of the above-described configuration will be described with reference to a flowchart shown in FIG. 18 as an example. As shown in FIG. 6, in a case where the operation program 80 is activated, the CPU 72 of the information processing apparatus 40 functions as the acquisition unit 85, the RW control unit 86, the instruction reception unit 87, the correction unit 88, the prediction unit 89, and the display control unit 90.

First, the acquisition unit 85 acquires the target Raman spectral data 37T from the Raman spectrometer 12 (step ST100). The target Raman spectral data 37T is output from the acquisition unit 85 to the RW control unit 86, and is stored in the storage 70 under the control of the RW control unit 86 (step ST110).

Under the control of the display control unit 90, the used flow cell selection screen 115 shown in FIG. 11 is displayed on the display 41 (step ST120). The user U selects the flow cell 11 used in a case of obtaining the target Raman spectral data 37T from the pull-down menu 116 and selects the OK button 117. As a result, as shown in FIG. 12, the designation information 120 is received by the instruction reception unit 87 (step ST130). The designation information 120 is output from the instruction reception unit 87 to the RW control unit 86.

The target Raman spectral data 37T is read out from the storage 70 under the control of the RW control unit 86. In addition, the correction coefficient CF corresponding to the flow cell 11 designated by the designation information 120 is acquired by being read out from the correction coefficient information 81 of the storage 70 (step ST140). The target Raman spectral data 37T and the correction coefficient CF are output from the RW control unit 86 to the correction unit 88.

In the correction unit 88, as shown in FIG. 13, the target Raman spectral data 37T is corrected with the correction coefficient CF to generate the corrected target Raman spectral data 37TC (step ST150). The corrected target Raman spectral data 37TC is output from the correction unit 88 to the prediction unit 89.

In the prediction unit 89, as shown in FIG. 14, the corrected target Raman spectral data 37TC is input to the antibody concentration prediction model 82, and the prediction result 95 is output from the antibody concentration prediction model 82 (step ST160). The prediction result 95 is output from the prediction unit 89 to the display control unit 90.

As shown in FIG. 17, under the control of the display control unit 90, the prediction result display screen 135 is displayed on the display 41 (step ST170), and the predicted value of the concentration of the antibody 16 of the prediction result 95 is made available for viewing by the user U.

The user U makes various decisions based on the predicted value displayed on the prediction result display screen 135. For example, a case where conditions such as the culture condition of the antibody-producing cells are set using the small-scale equipment is performed is considered. In this case, in a case where the predicted value is worse than a target value, the user U makes a decision to stop a current experiment and transition to an experiment based on a new condition. In addition, a case where the condition setting is completed and mass production is performed using the large-scale equipment is considered. In this case, in a case where the predicted value is worse than the target value, the user U makes a decision to interrupt the mass production and perform maintenance of the culture tank of the culture section 13 or the various chromatography devices of the purification section 14.

As described above, the information processing apparatus 40 comprises the acquisition unit 85, the RW control unit 86, the correction unit 88, and the prediction unit 89. The prediction unit 89 uses the antibody concentration prediction model 82 that has been calibrated with the standard Raman spectral data 37ST obtained from the standard suspension 18ST in the standard measurement environment (trained using the learning standard Raman spectral data 37STL). The acquisition unit 85 acquires the target Raman spectral data 37T obtained from the target suspension 18T in which the concentration of the antibody 16 is unknown, in the target measurement environment different from the standard measurement environment. The RW control unit 86 acquires the correction coefficient CF derived by comparing the standard Raman spectral data 37ST with the reference Raman spectral data 37R that corresponds to the target Raman spectral data 37T and that is obtained from the standard suspension 18ST in the target measurement environment. The correction unit 88 corrects the target Raman spectral data 37T with the correction coefficient CF to generate corrected target Raman spectral data 37TC. The prediction unit 89 applies the corrected target Raman spectral data 37TC to the antibody concentration prediction model 82 so that the antibody concentration prediction model 82 predicts the concentration of the antibody 16.

The difference in the Raman spectral data 37 resulting from the measurement environment can be eliminated by correcting the difference using the correction coefficient CF. Therefore, even in a case where the measurement environment of the Raman scattering light RSL is changed, it is possible to obtain a highly reliable prediction result 95 for the concentration of the antibody 16 in the target suspension 18T.

As shown in FIG. 10, the correction coefficient CF is derived based on a ratio of the intensity values of the standard Raman spectral data 37ST and the reference Raman spectral data 37R corresponding to the component in the standard suspension 18ST. Therefore, it is possible to easily derive a valid correction coefficient CF. In addition, a machine learning model that outputs the correction coefficient CF in response to the input of the standard Raman spectral data 37ST and the reference Raman spectral data 37R may be used.

In addition, as shown in FIG. 10, the correction coefficient CF is derived by excluding a ratio of the intensity values corresponding to the measuring device for the Raman scattering light RSL, in this case, the optical system 58 of the flow cell 11.

As described above, in a case where the flow cell 11 is used, not only the peak corresponding to the component in the target suspension 18T, but also a peak derived from the optical system 58 of the flow cell 11, is observed in the Raman spectral data 37. The peak derived from the optical system 58 appears differently from the peak corresponding to the component in the target suspension 18T. Therefore, as shown in the graph 111 in FIG. 10, the ratio of the intensity value of the standard Raman spectral data 37ST corresponding to the optical system 58 to the intensity value of the reference Raman spectral data 37R corresponding to the optical system 58 is a value that deviates from the ratio of the intensity value of the standard Raman spectral data 37ST corresponding to the component in the standard suspension 18ST to the intensity value of the reference Raman spectral data 37R corresponding to the component in the standard suspension 18ST. Therefore, in a case where the correction coefficient CF is derived by excluding the ratio of the intensity values corresponding to the optical system 58 of the flow cell 11, a more valid correction coefficient CF can be derived.

As shown in FIG. 8, the standard measurement environment and the target measurement environment differ in whether the measuring device for the Raman scattering light RSL, in this case the flow cell 11, is used. Therefore, a more valid correction coefficient CF can be derived.

As shown in FIG. 7, there are a plurality of the target measurement environments, and the plurality of target measurement environments use different types of the flow cells 11. In addition, the correction coefficient CF is stored in advance in the storage 70 for each of the plurality of target measurement environments. Then, as shown in FIGS. 11 and 12, the instruction reception unit 87 receives the designation of the type of the flow cell 11 used. The RW control unit 86 acquires the correction coefficient CF corresponding to the designation by reading out the correction coefficient CF from the storage 70.

Therefore, it is possible to accommodate the plurality of target measurement environments without any problem. In addition, it is possible to eliminate the need to prepare the antibody concentration prediction model 82 for each of the plurality of target measurement environments and hence for each of the plurality of types of flow cells 11. Then, as the supervised training data 130 of the antibody concentration prediction model 82, at least the learning standard Raman spectral data 37STL and the ground-truth antibody concentration 95CA obtained in the standard measurement environment are required. Therefore, the supervised training data 130 can be easily prepared.

The flow cells 11 differ in at least one of the distance D between the second surface 62 of the transparent plate 60 that is an incident surface of the Raman scattering light RSL of the optical system 58 and the wall surface 63A of the flow path 52 facing the second surface 62, the reflectivity R of the wall surface 63A, or the focal length FL of the optical system 58. It is possible to accommodate the change in the target measurement environment due to the difference in the distance D, the reflectivity R, and the focal length FL without any problem.

The concentration is the most popular indicator for understanding the physicochemical characteristics of the antibody 16. Therefore, in a case where the concentration is predicted as the state of the antibody 16 as in this example, the user U can easily understand the physicochemical characteristics of the antibody 16.

The biopharmaceuticals including the antibody 16 are called antibody drugs and are widely used to treat chronic diseases such as cancer, diabetes, and rheumatoid arthritis, as well as rare diseases such as hemophilia and Crohn's disease. Therefore, according to this example in which the protein is the antibody 16, it is possible to promote the development of antibody drugs widely used for the treatment of various diseases.

The Raman scattering light RSL is likely to reflect information derived from a functional group of an amino acid in a protein. Therefore, by using the electromagnetic waves as the Raman scattering light RSL as in this example, it is possible to acquire the Raman spectral data 37 that accurately reflects the physical properties such as the concentration of the antibody 16, which is a protein.

As shown in FIGS. 15 and 16, the antibody concentration prediction model 82 is a machine learning model that has been trained using the learning standard Raman spectral data 37STL as supervised training data. The machine learning model is generally used to predict unknown parameters, and the prediction accuracy can be increased to a certain level through learning. Therefore, it is possible to easily generate the antibody concentration prediction model 82 having a relatively high prediction accuracy.

### (Modification Example)

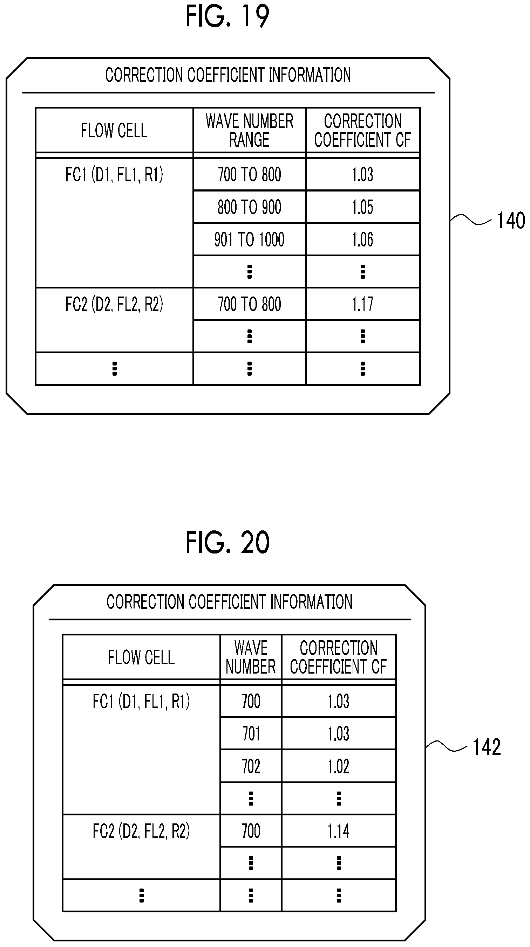

In the correction coefficient information 81 of the first embodiment, an example has been described in which one correction coefficient CF is registered for one flow cell 11, but the present disclosure is not limited to this. As shown in correction coefficient information 140 in FIG. 19 as an example, the correction coefficient CF may be registered for each wave number range of the Raman spectral data 37 for one flow cell 11. In addition, as shown in correction coefficient information 142 in FIG. 20 as an example, the correction coefficient CF may be registered for each wave number for one flow cell 11. In this way, by setting the correction coefficient CF finely for each wave number range, for each wave number, and the like, more precise correction can be performed on the target Raman spectral data 37T.

### [Second Embodiment]

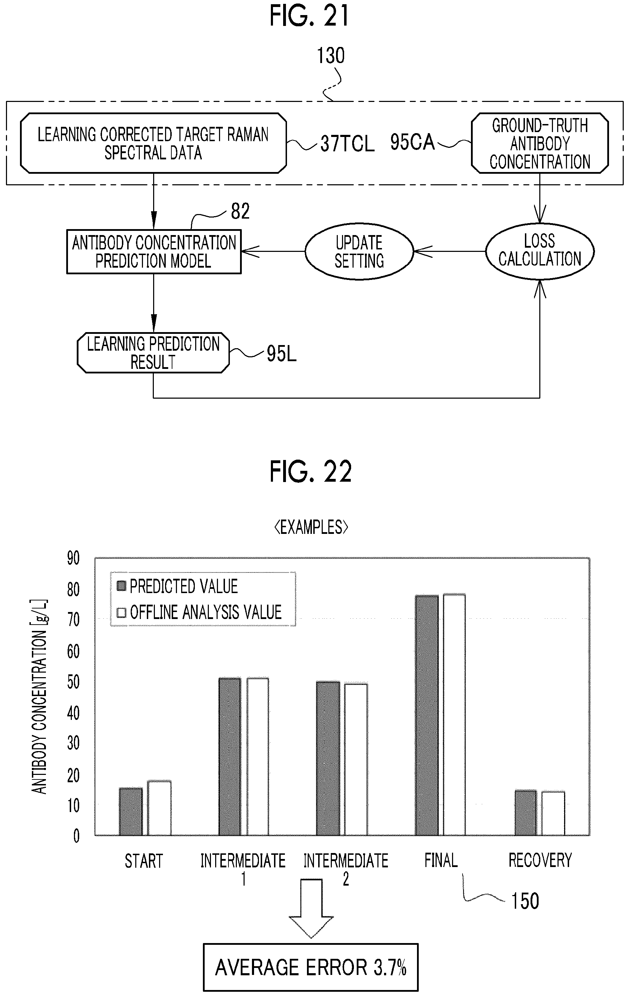

In the first embodiment, as shown in FIG. 16, the supervised training data 130 including the learning standard Raman spectral data 37STL has been described as an example, but the present disclosure is not limited to this. As shown in FIG. 21 as an example, in addition to the supervised training data 130 including the learning standard Raman spectral data 37STL, supervised training data 130 including learning corrected target Raman spectral data 37TCL may be used. The learning corrected target Raman spectral data 37TCL is generated by correcting the target Raman spectral data 37T measured in the past by the correction coefficient CF.

As described above, in a second embodiment, the antibody concentration prediction model 82 is trained using the learning corrected target Raman spectral data 37TCL. Therefore, a large amount of the supervised training data 130 can be secured. As a result, the prediction accuracy of the antibody concentration prediction model 82 can be improved.

### [Examples]

First, in the standard measurement environment in which the sensor unit 35 of the Raman spectrometer 12 is directly immersed in the standard suspension 18ST in the tank 100A shown in FIG. 8, the Raman scattering light RSL from the standard suspension 18ST was measured. As a result, the standard Raman spectral data 37ST was obtained. As shown in FIG. 16, the antibody concentration prediction model 82 was trained using the supervised training data 130 including the standard Raman spectral data 37ST obtained in this way as the learning standard Raman spectral data 37STL, and the antibody concentration prediction model 82 of which the prediction accuracy has reached the set level was obtained.

As shown in FIG. 1, the flow cell 11 was provided in the flow-out path 26 of the concentration section 15, the sensor unit 35 of the Raman spectrometer 12 was connected to the flow cell 11, and the Raman scattering light RSL of the target suspension 18T was measured, thereby obtaining the target Raman spectral data 37T. As shown in FIG. 13, the correction coefficient CF derived by the correction coefficient derivation processing 101 shown in FIG. 10 was applied to the target Raman spectral data 37T to generate the corrected target Raman spectral data 37TC.

As shown in FIG. 14, the corrected target Raman spectral data 37TC was input to the antibody concentration prediction model 82, and the prediction result 95 was output from the antibody concentration prediction model 82. On the other hand, the concentration of the antibody 16 in the target suspension 18T was measured using the mass spectrometry function of the HPLC device. The prediction of the concentration of the antibody 16 using the antibody concentration prediction model 82 and the actual measurement of the concentration of the antibody 16 using the HPLC device were performed at each of the following time points: the start of the concentration in the concentration section 15; an intermediate 1 point; an intermediate 2 point; a final point; and a point of recovery of a buffer solution for cleaning.

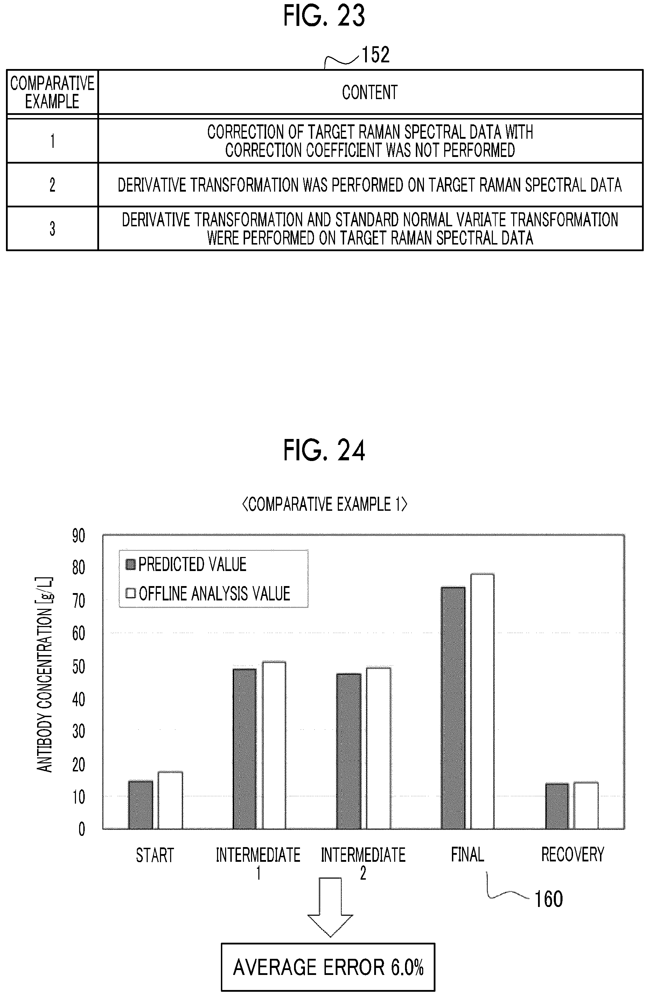

FIG. 22 shows a comparison graph 150 of a predicted value and an actual measurement value (in the drawing, referred to as an offline analysis value; the same applies to FIGS. 24 to 26) of the concentration of the antibody 16 at each time point in Examples, the predicted value being obtained by the antibody concentration prediction model 82 and the actual value being obtained by the HPLC device. An average value of errors of the predicted value with respect to the actual measurement value at each time point (hereinafter, referred to as an average error) was 3.7%.

### [Comparative Examples]

As shown in a table 152 of FIG. 23 as an example, Comparative Example 1, Comparative Example 2, and Comparative Example 3 were prepared. Comparative Example 1 is a case where the target Raman spectral data 37T was input to the antibody concentration prediction model 82 as it is without performing the correction of the target Raman spectral data 37T with the correction coefficient CF. Comparative Example 2 is a case where the derivative transformation (including 15-point data smoothing) disclosed in JP2022-552876A was performed on the target Raman spectral data 37T. Comparative Example 3 is a case where the derivative transformation (including 15-point data smoothing) and the standard normal variate transformation disclosed in JP2022-552876A were performed on the target Raman spectral data 37T.

FIG. 24 shows a comparison graph 160 of a predicted value and an actual measurement value of the concentration of the antibody 16 at each time point in Comparative Example 1, the predicted value being obtained by the antibody concentration prediction model 82 and the actual value being obtained by the HPLC device. At each time point, the predicted value lower than the actual measurement value is derived. This is consistent with a case where the correction coefficient CF shown in FIG. 10 is 1 or more. In this case, the average error was 6.0%, which was worse than that in Examples.

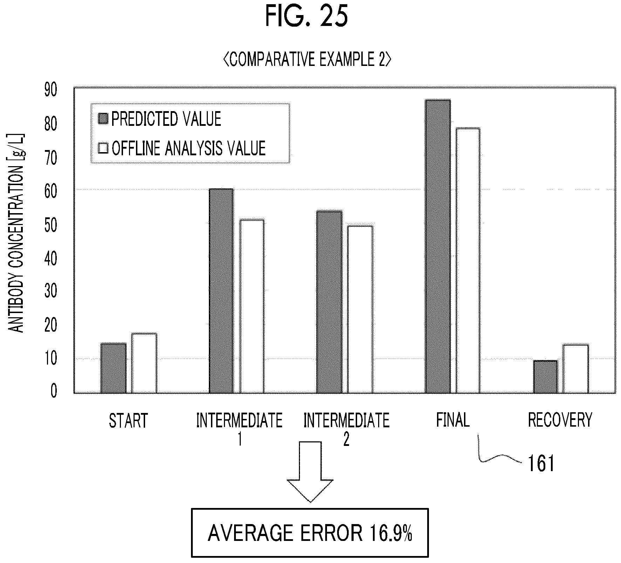

FIG. 25 shows a comparison graph 161 of a predicted value and an actual measurement value of the concentration of the antibody 16 at each time point in Comparative Example 2, the predicted value being obtained by the antibody concentration prediction model 82 and the actual value being obtained by the HPLC device. In this case, the average error was 16.9%, which was significantly worse than that in Examples.

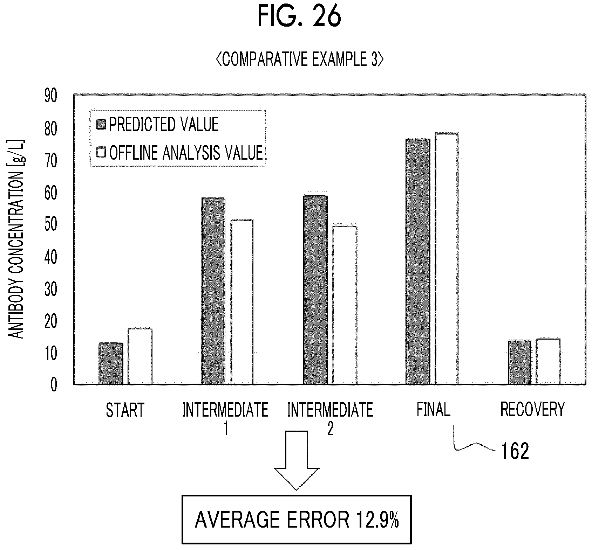

FIG. 26 shows a comparison graph 162 of a predicted value and an actual measurement value of the concentration of the antibody 16 at each time point in Comparative Example 3, the predicted value being obtained by the antibody concentration prediction model 82 and the actual value being obtained by the HPLC device. In this case, the average error was 12.9%, which was significantly worse than that in Examples, as in Comparative Example 1.

As described above, it was confirmed that Examples to which the technology of the present disclosure was applied have more useful effects than Comparative Examples 1 to 3.

The concentration of the antibody 16 is predicted as the state, but the present disclosure is not limited to this. The concentration of the aggregate 17 of the antibody 16 may be predicted. In addition, instead of or in addition to the concentration, the purity, the density, or the like may be predicted. The purity is calculated, for example, by using the sum of the amount of the antibody 16 and the amount of the impurities as a denominator, and using the amount of the antibody 16 as a numerator. For example, two or more states such as the concentration and the density may be predicted. Furthermore, the indicator is not limited to a quantitative indicator such as the concentration and the purity, and may be a qualitative indicator such as the level of the quality of the antibody 16 (two stages of good or bad or five stages of 1 to 5).

As other examples of the state, the state may be a viable cell density, a glucose concentration, a glutamic acid concentration, an amino acid concentration, a solvent component concentration, an additive concentration, a lactic acid concentration, an ammonia concentration, a concentration of cell-derived protein/cell-derived DNA, a concentration of other cell metabolites, an antibody fragment concentration, or a charge isomer concentration.

A substance for which the Raman spectral data 37 is measured is not limited to the antibody 16. The substance may be proteins, peptides, nucleic acid (DNA or ribonucleic acid (RNA)), lipids, viruses, virus subunits, a virus-like particles, and the like other than the antibody 16.

The component is not limited to the antibody 16. Examples of the component include cytokine (interferon, interleukin, or the like), hormone (insulin, glucagon, follicle-stimulating hormone, erythropoietin, or the like), a growth factor (insulin-like growth factor (IGF)-1, basic fibroblast growth factor (bFGF), or the like), a blood coagulation factor (seventh factor, eighth factor, ninth factor, or the like), an enzyme (lysosomal enzyme, deoxyribonucleic acid (DNA) degrading enzyme, or the like), a fragment crystallizable (Fc) fusion protein, a receptor, albumin, and a protein vaccine. In addition, examples of the antibody 16 include a bispecific antibody, an antibody-drug conjugate, a low-molecular-weight antibody, and a sugar-chain-modified antibody.

The electromagnetic waves are not limited to the Raman scattering light RSL, and thus the spectral data is not limited to the Raman spectral data 37. The physical property data may be infrared absorption spectral data, near infrared absorption spectrum data, nuclear magnetic resonance spectrum data, ultraviolet visible absorption spectroscopy (UV-Vis) spectrum data, or fluorescence spectrum data.

The antibody concentration prediction model 82 is not limited to the machine learning model. A model generated by a multivariate analysis or a statistical analysis may be used. Examples of the multivariate analysis and the statistical analysis include linear regression, multiple regression, principal component regression, partial least squares regression, logistic regression, Lasso regression, ridge regression, support vector regression, and Gaussian process regression. In the model generated by such multivariate analysis and statistical analysis, determining a coefficient of a regression equation based on at least two pieces of the supervised training data 130 is an example of "calibration" according to the technology of the present disclosure.

In addition, the different measurement environments include a case where the measurement environments differ in the age of an optical component, the shape of the flow path 52, the temperature, the humidity, the flow rate and the flow velocity of the target suspension 18T, or the like, in addition to or instead of a case where the measurement environments differ in whether the flow cell 11 is used or the type of the flow cell 11. Specifically, the optical component is an optical fiber that is disposed in the sensor unit 35 of the Raman spectrometer 12 and that guides the excitation light EL and the Raman scattering light RSL, a light source of the excitation light EL, the optical system 58, or the like. The shape of the flow path 52 specifically refers to a cross-sectional shape of the flow path 52, such as a circular cross-sectional shape or an elliptical cross-sectional shape, or an overall shape of the flow path 52, such as a straight, U-shaped, or L-shaped shape.

The flow path 52 of the flow cell 11 may have a U-shape. In addition, the shape of the flow cell 11 is not limited to the cylindrical shape, and may be a rectangular cylindrical shape. The cross-sectional shape of the flow path 52 is also not limited to the circular shape, and may be an elliptical shape or a rectangular shape. In addition, the flow cell 11 may be formed of a composite material such as a carbon fiber reinforced resin.

The fluid is not limited to the purified solution 18 and the mixed solution 18M of the purified solution 18 and the concentrated solution. The fluid may be a cell culture solution before being subjected to cell removal by the cell removal filter in the culture section 13, or may be a culture supernatant after the cell removal. The fluid may be a cell culture solution (medium) that does not contain the biological molecules before being supplied to the culture section 13. The fluid may be a purified solution introduced into the various chromatography devices or a purified solution sent from the various chromatography devices. The fluid may be a purified solution after a process of inactivating the virus is completed.

The fluid is not limited to one used for manufacturing the biopharmaceuticals, and may be, for example, river water collected for investigating water pollution. In addition, the fluid is not limited to liquid, and may be gas.

In each of the above-described embodiments, so-called in-line sensing has been exemplified in which a measurement sensor, in this case the sensor unit 35 of the Raman spectrometer 12, is installed within the manufacturing process to perform measurements within the manufacturing process, but the present disclosure is not limited to this. The technology of the present disclosure may also be applied to offline sensing in which measurements are performed outside the manufacturing process using a measurement sensor that is separate from the manufacturing process.

The optical element constituting the optical system 58 is not limited to the exemplary ball lens 59. The optical element may be a hemispherical lens, a plano-convex lens, a biconvex lens, a cylindrical lens, or the like. In addition, the optical element is not limited to the exemplary disk-shaped transparent plate 60 having the first surface 61 and the second surface 62 that are parallel to each other. The optical element may be a transparent plate having a first surface having a shape following a shape of an emission surface of the ball lens, the plano-convex lens, the biconvex lens, or the like, and a planar second surface. The transparent plate may not be necessary, and the optical system 58 may be composed of only the ball lens 59, the hemispherical lens, or the like.

The information processing apparatus 40 may be a personal computer that is installed in the pharmaceutical facility as shown in FIG. 1 or may be a server computer that is installed in a data center independent of the pharmaceutical facility.

In a case where the information processing apparatus 40 is configure by the server computer, the target Raman spectral data 37T is transmitted from the personal computer installed in each pharmaceutical facility to the server computer via a network such as the Internet. The server computer delivers various screens such as the used flow cell selection screen 115 and the prediction result display screen 135 to the personal computer in a format of screen data for web distribution created by a markup language such as Extensible Markup Language (XML). The personal computer reproduces a screen displayed on a web browser based on the screen data and displays the reproduced screen on the display. Note that, instead of XML, another data description language, such as Javascript (registered trademark) Object Notation (JSON), may be used.

The hardware configuration of the computer constituting the information processing apparatus 40 according to the technology of the present disclosure can be modified in various ways. For example, the information processing apparatus 40 may be configured by a plurality of computers separated as hardware in order to improve processing capacity and reliability. For example, the function of the correction unit 88 and the function of the prediction unit 89 may be distributed to two computers. In such a case, the information processing apparatus 40 is configured by the two computers.

As described above, the hardware configuration of the computer of the information processing apparatus 40 can be changed as appropriate in accordance with required performance, such as processing capacity, safety, and reliability. Further, it goes without saying that, in addition to the hardware, an application program such as the operation program 80 can be duplicated or distributed and stored in a plurality of storages for the purpose of securing the safety and the reliability.

In each of the above-described embodiments, for example, the following various processors can be used as a hardware structure of processing units executing various processes, such as the acquisition unit 85, the RW control unit 86, the instruction reception unit 87, the correction unit 88, the prediction unit 89, and the display control unit 90. As described above, the various processors include, in addition to the CPU 72 that is a general-purpose processor which executes software (operation program 80) to function as various processing units, a programmable logic device (PLD) that is a processor of which a circuit configuration can be changed after manufacture, such as a field programmable gate array (FPGA), and a dedicated electrical circuit that is a processor having a circuit configuration which is designed for exclusive use to execute specific processing, such as an application specific integrated circuit (ASIC).

One processing unit may be configured of one of the various processors or may be configured of a combination of two or more processors of the same type or different types (for example, a combination of a plurality of FPGAs and/or a combination of a CPU and an FPGA). In addition, a plurality of processing units may be configured of one processor.

As an example of configuring the plurality of processing units with one processor, first, there is a form in which, as typified by computers such as a client and a server, one processor is configured of a combination of one or more CPUs and software and the processor functions as the plurality of processing units. Second, there is a form in which, as typified by a system on chip (SoC) and the like, a processor that implements functions of an entire system including the plurality of processing units with one integrated circuit (IC) chip is used. As described above, various processing units are configured by using one or more of the various processors as a hardware structure.

In addition, more specifically, an electric circuit (circuitry) in which circuit elements, such as semiconductor elements, are combined can be used as the hardware structure of these various processors.

It is possible to understand the technologies described in the following supplementary notes from the above description.

### [Supplementary Note 1]

An information processing apparatus that predicts a state of a target component in a suspension in which biological molecules are dispersed as components in a liquid, based on spectral data obtained by measuring electromagnetic waves emitted from the suspension, the information processing apparatus comprising:
a processor,
in which the processor is configured to
   use a state prediction model calibrated with standard spectral data obtained from a standard suspension in a standard measurement environment,
   acquire target spectral data obtained from a target suspension in which the state of the target component is unknown, in a target measurement environment different from the standard measurement environment,
   acquire a correction coefficient derived by comparing the standard spectral data with reference spectral data that corresponds to the target spectral data and that is obtained from the standard suspension in the target measurement environment,
   correct the target spectral data with the correction coefficient to generate corrected target spectral data, and
   apply the corrected target spectral data to the state prediction model so that the state prediction model predicts the state of the target component.

### [Supplementary Note 2]

The information processing apparatus according to Supplementary Note 1,
in which the correction coefficient is derived based on a ratio of intensity values of the standard spectral data and the reference spectral data corresponding to the component in the standard suspension.

### [Supplementary Note 3]

The information processing apparatus according to Supplementary Note 1 or 2,
in which the correction coefficient is derived by excluding a ratio of intensity values corresponding to a measuring device for the electromagnetic waves.

### [Supplementary Note 4]

The information processing apparatus according to Supplementary Note 3,
in which the measuring device is a flow cell having a flow path through which the suspension flows and an optical system for introducing the electromagnetic waves, and
the intensity value corresponding to the measuring device is an intensity value corresponding to the optical system.

### [Supplementary Note 5]

The information processing apparatus according to any one of Supplementary Notes 1 to 4,
in which the standard measurement environment and the target measurement environment differ in any of whether a measuring device for the electromagnetic waves is used or a type of the measuring device.

### [Supplementary Note 6]

The information processing apparatus according to Supplementary Note 5,
in which the measuring device is a flow cell having a flow path through which the suspension flows and an optical system for introducing the electromagnetic waves.

### [Supplementary Note 7]

The information processing apparatus according to Supplementary Note 6,
in which there are a plurality of the target measurement environments, and
the plurality of target measurement environments use different types of the flow cells.

### [Supplementary Note 8]

The information processing apparatus according to Supplementary Note 7,
in which the correction coefficient is stored in advance in a storage unit for each of the plurality of target measurement environments, and
the processor is configured to
   receive a designation of the type of the flow cell used, and
   acquire the correction coefficient corresponding to the designation by reading out the correction coefficient from the storage unit.

### [Supplementary Note 9]

The information processing apparatus according to Supplementary Note 7 or 8,
in which the flow cells differ in at least one of a distance between an incident surface of the optical system for the electromagnetic waves and a wall surface of the flow path facing the incident surface, a reflectivity of the wall surface, or a focal length of the optical system.

### [Supplementary Note 10]

The information processing apparatus according to any one of Supplementary Notes 1 to 9,
in which the state prediction model is calibrated with the corrected target spectral data.

### [Supplementary Note 11]

The information processing apparatus according to any one of Supplementary Notes 1 to 10,
in which the state of the target component is a concentration of the target component.

### [Supplementary Note 12]

The information processing apparatus according to any one of Supplementary Notes 1 to 11,
in which the target component is a protein.

### [Supplementary Note 13]

The information processing apparatus according to Supplementary Note 12,
in which the protein is an antibody.

### [Supplementary Note 14]

The information processing apparatus according to any one of Supplementary Notes 1 to 13,
in which the electromagnetic waves are Raman scattering light.

### [Supplementary Note 15]

The information processing apparatus according to any one of Supplementary Notes 1 to 14,
in which the state prediction model is a machine learning model that has been trained using the standard spectral data as supervised training data.

In the technology of the present disclosure, the above-described various embodiments and/or various modification examples may be combined with each other as appropriate. In addition, it is needless to say that the present disclosure is not limited to each of the above-described embodiments, and various configurations can be used without departing from the gist of the present disclosure. Furthermore, the technology of the present disclosure extends to a storage medium that non-transitorily stores the program, and a computer program product including the program, in addition to the program.

The above descriptions and illustrations are detailed descriptions of portions related to the technology of the present disclosure and are merely examples of the technology of the present disclosure. For example, description related to the above configurations, functions, actions, and effects is description related to an example of configurations, functions, actions, and effects of the parts according to the technology of the present disclosure. Thus, it goes without saying that unnecessary portions may be deleted, new elements may be added, or replacement may be made to the content of the above description and the content of the drawings without departing from the gist of the technique of the present disclosure. Further, in order to avoid complications and facilitate understanding of the parts related to the technology of the present disclosure, descriptions of common general knowledge and the like that do not require special descriptions for enabling the implementation of the technology of the present disclosure are omitted, in the contents described and shown above.

In the present specification, the term "A and/or B" is synonymous with the term "at least one of A or B". That is, the term "A and/or B" means only A, only B, or a combination of A and B. In addition, in the present specification, the same approach as "A and/or B" is applied to a case in which three or more matters are represented by connecting the matters with "and/or".

All documents, patent applications, and technical standards mentioned in the present specification are incorporated herein by reference to the same extent as in a case in which each document, each patent application, and each technical standard are specifically and individually described by being incorporated by reference.

## Claims

1. An information processing apparatus that predicts a state of a target component in a suspension in which biological molecules are dispersed as components in a liquid, based on spectral data obtained by measuring electromagnetic waves emitted from the suspension, the information processing apparatus comprising:
a processor,
wherein the processor is configured to
use a state prediction model calibrated with standard spectral data obtained from a standard suspension in a standard measurement environment,
acquire target spectral data obtained from a target suspension in which the state of the target component is unknown, in a target measurement environment different from the standard measurement environment,
acquire a correction coefficient derived by comparing the standard spectral data with reference spectral data that corresponds to the target spectral data and that is obtained from the standard suspension in the target measurement environment,
correct the target spectral data with the correction coefficient to generate corrected target spectral data, and
apply the corrected target spectral data to the state prediction model so that the state prediction model predicts the state of the target component.

2. The information processing apparatus according to claim 1,
wherein the correction coefficient is derived based on a ratio of intensity values of the standard spectral data and the reference spectral data corresponding to the component in the standard suspension.

3. The information processing apparatus according to claim 1,
wherein the correction coefficient is derived by excluding a ratio of intensity values corresponding to a measuring device for the electromagnetic waves.

4. The information processing apparatus according to claim 3,
wherein the measuring device is a flow cell having a flow path through which the suspension flows and an optical system for introducing the electromagnetic waves, and
the intensity value corresponding to the measuring device is an intensity value corresponding to the optical system.

5. The information processing apparatus according to claim 1,
wherein the standard measurement environment and the target measurement environment differ in any of whether a measuring device for the electromagnetic waves is used or a type of the measuring device.

6. The information processing apparatus according to claim 5,
wherein the measuring device is a flow cell having a flow path through which the suspension flows and an optical system for introducing the electromagnetic waves.

7. The information processing apparatus according to claim 6,
wherein there are a plurality of the target measurement environments, and
the plurality of target measurement environments use different types of the flow cells.

8. The information processing apparatus according to claim 7,
wherein the correction coefficient is stored in advance in a storage unit for each of the plurality of target measurement environments, and
the processor is configured to
receive a designation of the type of the flow cell used, and
acquire the correction coefficient corresponding to the designation by reading out the correction coefficient from the storage unit.

9. The information processing apparatus according to claim 7,
wherein the flow cells differ in at least one of a distance between an incident surface of the optical system for the electromagnetic waves and a wall surface of the flow path facing the incident surface, a reflectivity of the wall surface, or a focal length of the optical system.

10. The information processing apparatus according to claim 1,
wherein the state prediction model is calibrated with the corrected target spectral data.

11. The information processing apparatus according to claim 1,
wherein the state of the target component is a concentration of the target component.

12. The information processing apparatus according to claim 1,
wherein the target component is a protein.

13. The information processing apparatus according to claim 12,
wherein the protein is an antibody.

14. The information processing apparatus according to claim 1,
wherein the electromagnetic waves are Raman scattering light.

15. The information processing apparatus according to claim 1,
wherein the state prediction model is a machine learning model that has been trained using the standard spectral data as supervised training data.

16. An operation method of an information processing apparatus that predicts a state of a target component in a suspension in which biological molecules are dispersed as components in a liquid, based on spectral data obtained by measuring electromagnetic waves emitted from the suspension, the operation method comprising:
using a state prediction model calibrated with standard spectral data obtained from a standard suspension in a standard measurement environment;
acquiring target spectral data obtained from a target suspension in which the state of the target component is unknown, in a target measurement environment different from the standard measurement environment;
acquiring a correction coefficient derived by comparing the standard spectral data with reference spectral data that corresponds to the target spectral data and that is obtained from the standard suspension in the target measurement environment;
correcting the target spectral data with the correction coefficient to generate corrected target spectral data; and
applying the corrected target spectral data to the state prediction model so that the state prediction model predicts the state of the target component.

17. An operation program of an information processing apparatus that predicts a state of a target component in a suspension in which biological molecules are dispersed as components in a liquid, based on spectral data obtained by measuring electromagnetic waves emitted from the suspension, the operation program causing a computer to execute a process comprising:
using a state prediction model calibrated with standard spectral data obtained from a standard suspension in a standard measurement environment;
acquiring target spectral data obtained from a target suspension in which the state of the target component is unknown, in a target measurement environment different from the standard measurement environment;
acquiring a correction coefficient derived by comparing the standard spectral data with reference spectral data that corresponds to the target spectral data and that is obtained from the standard suspension in the target measurement environment;
correcting the target spectral data with the correction coefficient to generate corrected target spectral data; and
applying the corrected target spectral data to the state prediction model so that the state prediction model predicts the state of the target component.
